# EUROPEAN PATENT APPLICATION

(11) **EP 3 797 747 A1**
(43) Date of publication of application: **31.03.2021**
(21) Application number: 19200642.7
(22) Date of filing: 30.09.2019
(51) Int. Cl.: A61F 13/551, A61F 13/58, A61F 13/84, A61F 13/56

(54) **DISPOSAL TAB, METHOD OF MAKING A DISPOSAL TAB, DISPOSAL TAPE AND ABSORBENT ARTICLE WITH DISPOSAL TAB**

(71) Applicant: 3M Innovative Properties Company, Saint Paul, MN 55133-3427 (US)
(72) Inventor: GÜLER, Serhat, 59860 Corlu/Tekirdag (TR); GENC, Yavuz, Corlu, Tekirdag (TR); ABAKAY, Ayca, 595850 Corlu/Tekirdag (TR); CORUMLUOGLU, Evren, 34805 Kavacik/Istanbul (TR)
(74) Representative: Vollmers, Hans-Gerd

(57) **Abstract**

The present disclosure relates to a disposal tab 10, 10', 11, 11', 12, 12', 13, 14, 15, 15', 16, 16', 17 for securing a disposable article 200 in a disposal configuration comprising a first and second tab element 20, 20', 30, 30' and to a method of making such a disposal tab 10, 10', 11, 11', 12, 12', 13, 14, 15, 15', 16, 16', 17. The present disclosure further relates to a disposal tab tape 110 from which disposal tabs 10, 10', 11, 11', 12, 12', 13, 14, 15, 15', 16, 16', 17 according to the present disclosure can be cut as well as to a disposable article 200 comprising such a disposal tab 10, 10', 11, 11', 12, 12', 13, 14, 15, 15', 16, 16', 17.

## Description

The present disclosure relates to a disposal tab for securing a disposable article in a disposal configuration, to a disposal tape from which such disposal tabs can be cut as well as to a method of making such disposal tabs and to a disposable article having such disposal tabs.

Disposable pants or shorts infant style diapers or adult incontinence articles of this type are generally comparable to ordinary underwear pants and do not comprise closure or fastening tabs like open style disposable diapers. When such pants style infant diapers have been used and got soiled there is a desire to compact the absorbent article before final disposal. A disposable configuration is understood to be the configuration after use and - in particular - after the article has been soiled, when it is rolled up and compacted so that it is ready for disposal and/or can be thrown away. Since the pants style diapers or incontinence articles do not have closure or fastening tabs which could be used to compact the diaper or incontinence article before final disposal a separate so-called disposal tape has been proposed.

For example, EP 0 623 330 A2 (KAO, Hayase et al.) discloses a disposable diaper of the shorts type including a liquid permeable top sheet, a liquid impermeable back sheet, and an absorber interposed between the top sheet and the back sheet. Front and rear waist portions are connected together at opposite sides thereof, respectively. The diaper furthermore comprises a tape fastener adapted to fasten the diaper when the diaper is to be discarded. This tape fastener is disposed at a central section of an outer surface of the back sheet and extends in the longitudinal direction of the back sheet. This fastening tape is provided with a single fold or a Z-fold in order to provide a fastening tab of sufficient length to close the diaper in its compacted shape when it is to be discarded. The tape of the tape fastener may be formed of an elastic member.

Another disposable tape is described in WO 2007/032965A (3M, Dahm et al.) which relates to a laminate disposal tape tab comprising adjacent first and second tape tab elements, each having a first face and a second face. Preferably, the first face of each tape tab element is at least partially coated with a pressure-sensitive adhesive. A plastically deformable low yield extendible film having two end portions and an intermediate portion provided therebetween is attached to the first face of each of the first and second tape tab elements. A blanking film is preferably attached to the first face of each of the first and second tape tab elements and interposed between the tape tab elements and the low yield extendible film such that the low yield extendible film is not in contact with the adhesive coating of the tape tab elements at its intermediate portion.

The above-mentioned solutions offer an extensibility that is appreciated by consumers since it allows easy wrapping of the soiled disposable product. This extensibility is provided by single-fold or zig-zag fold constructions, in some cases having plastically deformable or elastically stretchable tape backings. On the one hand, these constructions do not provide a retraction force helping to keep the article in the disposal configuration. On the other hand, attempts with elastic backings have the disadvantage that the manufacturing costs are comparably high due to the relatively large elastic material consumption.

It is therefore a need to provide a disposal tab with improved functionality and which is widely usable with disposable articles for disposal. In particular, there is a need for a disposal tab with improved disposal functionality being producible at reasonable manufacturing costs.

The present disclosure relates to a disposal tab for securing a disposable article in a disposal configuration. The tab comprises a first tab element for attaching the tab onto a disposable article and a second tab element configured and adapted to elongate the tab along its major longitudinal extension. The second tab element comprises two inelastic portions and an intermediate elastic portion therebetween. The first and second tab elements are connected to each other through a non-releasable connection and - before use - through a releasable connection by arranging the second tab element on the first tab element, whereby a folded configuration is formed. The non-releasable connection is formed either by one end of the first or second tab element being folded onto itself and attached to the other tab element or by a c-shaped center strip encompassing an end of at least the first and second tab element. Before use - the second tab element is detachable from the first tab element upon use of the tab thereby releasing the folded configuration, while the tab elements are still connected through the non-releasable connection.

Such a disposal tab is advantageous because it improves the disposal functionality by providing a retraction force due to the intermediate elastic portion which can be manufactured at reasonable costs as well as by providing a reasonable elongation of the disposal tab in order to reach the other part of the disposable article to which the disposal tab may be attached being in a disposal configuration. This gives further flexibility where to arrange the disposal tab on a disposable article, i. e. even further spaced from an edge thereof. In particular, only the intermediate elastic portion instead of the entire second tab element being elastically stretchable reduces the need of elastic material still rendering the disposal tab elastic resulting in reduced material costs overall.

It is understood by the skilled person that a non-releasable connection withstands the forces as typically applied the disposal tab by a user of a disposable article to which the disposal tab may be attached. In contrast thereto, a releasable connection disconnects if such typical forces are applied to the disposal tab.

Typically, the non-releasable connection between the first and second tab elements is substantially located on one end of the disposal tab, i. e. connecting one end of the first and second tab element, whereas the releasable connection between the first and second tab element is substantially located at the other end, opposite to the end with the non-releasable connection, i. e. connecting the other end of the first and second tab elements. In some embodiments, the releasable connection may extend over a part or all of the extension of the disposal tab, whereas the non-releasable connection is usually located substantially at one end only.

A disposal tab according to the present disclosure may have a rectangular shape having a machine direction and a direction perpendicular thereto, i. e. cross direction. Typically, the disposal tab including the first tab element as well as the second tab element has a larger extension in cross direction than in machine direction. The first and second tab elements may have the same shape and/or size or may each have different shape and/or size. That is, the second tab element may extend beyond the first tab element or vice versa in one or more directions. For example, the cross directional extension may be between 30 and 90 mm and the machine directional extension may be between 5 and 50 mm.

In some embodiments, the first and second tab element with the first and second inelastic portions and the intermediate elastic portion are not folded, i. e. these are in a substantially flat configuration except for an end of the first and/or second tab element which may be folded to expose an adhesive present thereon to provide a non-releasable connection between the first and second tab element. Such a substantially flat configuration has the advantage that the overall thickness of the disposal tab can be kept reasonably low which may have advantages during manufacture (web handling) and use (disposal tab being comparably flat on a diaper surface to which the disposal tab may be attached).

The present disclosure moreover relates to a disposal tape from which disposal tabs according to the present disclosure can be cut. Optionally, the disposal tape is wound up into a roll of disposal tape. The disposal tape comprises a plurality of disposal tabs according to the present disclosure in an endless form and has a main longitudinal extension defining a machine direction. Such a disposal tape provides an easy and reliable way of a tape for making disposal tabs therefrom. Winding up into a roll provides for an efficient and reliable storage of the disposal tape. The roll of tape can easily and reliably be unwound to provide a disposal tape for making disposal tabs therefrom.

The present disclosure furthermore relates to a method of making a disposal tab according to the present disclosure. The method comprising the steps of providing a disposal tape according to the present disclosure comprising a plurality of disposal tabs according to the present disclosure in an endless form and having a main longitudinal extension defining a machine direction, optionally including the step of unwinding the disposal tape from a roll of disposal tape along its main longitudinal extension. The method further comprises the step of cutting the disposal tape in a direction perpendicular to the machine direction so as to obtain individual disposal tabs. Such a method is advantageous because it represents an easy and cost-efficient way of producing such disposal tabs.

The present disclosure also relates to a disposable article comprising a disposal tab according to the present disclosure attached to the outer surface of the disposable article. A disposable article with such a disposal tab thereon is advantageous because it improves the disposal functionality by providing a retraction force due to the intermediate elastic portion which can be manufactured at reasonable costs as well as by providing a reasonable elongation of the disposal tab in order to reach the other part of the disposable article to which the disposal tab may be attached being in a disposal configuration. This gives further flexibility where to arrange the disposal tab on a disposable article, i. e. even further spaced from an edge thereof. In particular, only the intermediate elastic portion instead of the entire second tab element being elastically stretchable reduces the need of elastic material still rendering the disposal tab elastic resulting in reduced material costs overall.

The present disclosure moreover relates to a disposal tab for securing a disposable article in a disposal configuration. The tab comprises a first tab element for attaching the tab onto a disposable article and a second tab element configured and adapted to elongate the tab along its major longitudinal extension. The second tab element comprises two inelastic portions and an intermediate elastic portion therebetween. The first and second tab elements are connected to each other through a third tab element providing a non-releasable connection between the first and third tab element and between the second and third tab element and - before use - through a releasable connection by arranging the second tab element on the third tab element and by arranging the second tab element on the third tab element, whereby a z-folded configuration is formed. The non-releasable connection is formed either by one end of the first and second tab element being folded onto itself and attached to the second and third tab element or by a c-shaped center strip encompassing an end of at least the first, second and/or third tab element. Before use - the second tab element is detachable from the third tab element and the third tab element is detachable from the first tab element upon use of the tab thereby releasing the z-folded configuration, while the tab elements are still connected through the non-releasable connection. Such a disposal tab is advantageous because it improves the disposal functionality by providing a retraction force due to the intermediate elastic portion which can be manufactured at reasonable costs as well as by providing a reasonable elongation of the disposal tab in order to reach the other part of the disposable article to which the disposal tab may be attached being in a disposal configuration. This gives further flexibility where to arrange the disposal tab on a disposable article, i. e. even further spaced from an edge thereof. In particular, only the intermediate elastic portion instead of the entire second tab element being elastically stretchable reduces the need of elastic material still rendering the disposal tab elastic resulting in reduced material costs overall. It is to be noted that the dimensions, materials and other properties or components, which are mentioned for a disposal tab having just a first and a second tab element, also apply to such a disposal tab having a third tab element.

In one embodiment, the c-shaped center strip of the disposal tab encompassing an end of each of the first and second tab element. The advantage of such a center strip is that additional manufacturing steps like folding can be avoided for the first tab element and the material for the center strip may be cheaper and thus may have cost advantages over using excess material of the first tab element. This applies to disposal tabs having first and second tab elements as well as to disposal tabs having first, second and third tab elements.

In one embodiment, the non-releasable connection of the disposal tab is made by means of adhesive bonding, thermobonding, ultrasonic bonding and/or cold pressure bonding. The bonding strength of such a non-releasable bonding should be higher than that of the releasable connection. An adhesive bonding provides for a reliable and easy to use bonding even of non-thermoplastic materials, whereas thermobonding and ultrasonic bonding - although requiring thermoplastic material - provides for a reliable bonding without extra material such as an adhesive. Cold pressure bonding is advantageous as neither heat nor another material is required. Typically, the releasable connection of the disposal tab may be made by means of adhesive bonding, thermobonding, ultrasonic bonding and/or cold pressure bonding. The bonding strength of such a releasable bonding should be lower than that of the non-releasable connection such that the releasable connection is easily separable by a user of disposable articles having such disposal tabs while the non-releasable connection is still present. An adhesive bonding provides for a reliable and easy to use bonding even of non-thermoplastic materials, whereas thermobonding and ultrasonic bonding - although requiring thermoplastic material - provides for a reliable bonding without extra material such as an adhesive. Cold pressure bonding is advantageous as neither heat nor another material is required. It is generally understood by the skilled person that the non-releasable connection is stronger and withstands higher pulling forces compared to the releasable connection. In other words, when the releasable connection is separated, the non-releasable connection still connects the tab elements.

In yet another embodiment, the first tab portion of the disposal tab comprises an adhesive on one major surface for permanently or non-releasably attaching the tab onto a disposable article. Putting an adhesive on one of the major surfaces may be advantageous because the disposal tab may easily and reliably be attachable to the outer surface of a disposable article using an adhesive.

In a further embodiment, an end of the first tab element of the disposal tab is folded onto itself and attached to the second tab element of the disposal tab such that an extension of the second tab element is formed which protrudes beyond the first tab element and which is attachable to the disposable article. Such an extension may be useful to increase the bonding surface and bonding strength between the disposal tab and the outer surface of a disposable article, to which the disposal tab is attachable. The extension bonds the second tab element directly to the disposable article and helps to prevent lifting of the first tab element when a pulling force is introduced into the disposal tab upon use.

In yet a further embodiment, the first and second tab element of the disposal tab comprise an adhesive on one of their major surfaces. The use of adhesives on the first and second tab element, in particular on the first tab element, is advantageous as the adhesive is useful in permanently or non-releasably attaching the disposal tab to the outer surface of a disposable article in an easy and reliable way. Also, the non-releasable connection between the first and second tab element can easily and reliably be formed using adhesives.

In still a further embodiment, the ends of the two inelastic portions of the second tab element of the disposal tab are spaced apart from each other. An exposed area of the intermediate elastic portion is thereby formed. Such spaced configuration of the inelastic portions is advantageous because thickened mid part of a tape comprising a plurality of such disposal tabs is avoided, whereby a roll of such tape is more stable. Also, less material of the inelastic portions is needed.

In yet a further embodiment, an anti-adhesive means such as a low adhesion backside is arranged on the intermediate elastic portion of the second tab element of the disposal tab. In particular, such an anti-adhesive means is present on an exposed area of the intermediate elastic portion present between the first and second inelastic portions of the second tab element. The anti-adhesive means may be an anti-adhesive coating or a film having an anti-adhesive surface which is exposed. The skilled person understands anti-adhesive such that an adhesive being in contact therewith may easily be detached therefrom without any damage. Such an anti-adhesive means has the advantage to allow easy detachment of an adhesive therefrom without blocking or damage. Such adhesive contact to the intermediate elastic portion may occur if a disposal tape comprising a plurality of disposal tabs according to the present disclosure is wound up into a roll such that several layers are above each other bringing an adhesive present on the first tab element into contact to the intermediate elastic portion. Also, the use of adhesive on the second tab element provides - together with an anti-adhesive means on the first tab element - for a releasable connection between the first and second tab element in an easy and reliable way.

In one embodiment, the ends of the two inelastic portions of the second tab element of the disposal tab abut each other. With such an abutting configuration of the inelastic portions, the intermediate elastic portion is prevented from coming in contact with an adhesive present on the inelastic portions as the inelastic portions cover the intermediate elastic portion. Thereby, no anti-adhesive means such as e. g. a low adhesion backside, is required to prevent the intermediate elastic portion from blocking with an adhesive.

In another embodiment, the intermediate elastic portion of the second tab element of the disposal tape is connected to each of the two inelastic portions by means of adhesive bonding, thermobonding, ultrasonic bonding and/or cold pressure bonding. An adhesive bonding provides for a reliable and easy to use bonding even of non-thermoplastic materials, whereas thermobonding and ultrasonic bonding - although requiring thermoplastic material - provides for a reliable bonding without extra material such as an adhesive. Cold pressure bonding is advantageous as neither heat nor another material is required.

In a further embodiment, a cover layer comprising cuts therein is arranged on the major surface of the intermediate elastic portion of the second tab element the disposal tab. The cover layer may face the adhesive of the second tab element. Such cover layer is advantageous as it functions as anti-adhesive means having e. g. a low adhesion backside thereon and thus prevents the intermediate elastic portion from blocking with an adhesive present of the inelastic portions.

In yet a further embodiment, the second tab element of the disposal tab comprises a backing having cuts therein and wherein the cover layer is formed by the backing of the second tab element. Such cuts may, for example, extend in machine direction of the disposal tab. The cuts may also extend obliquely or even perpendicular to the machine direction of the disposal tab. Furthermore, the cuts may be formed by slits, incisions, cut-outs, holes or punched portions of various shapes as described in, for example, EP 0 996 402 B1 (3M, Günther et al.) or EP 0 891 760 A1 (3M, Günther et al.). The cuts or slits may, for example, be made by knifes, rotary wheels, laser, punching tools or the like. Such cover layer is advantageous as it functions as anti-adhesive means having e. g. a low adhesion backside thereon and thus prevents the intermediate elastic portion from blocking with an adhesive present of the inelastic portions. In particular, forming the cover layer from the backing of the second tab element is beneficial as no extra or different material is required. Furthermore, arranging cuts in the cover layer allows for elongation of the cover layer and thus maintains the elasticity of the intermediate elastic portion to which the cover layer is attached.

In another embodiment, a blanking film is arranged between the adhesive of the second tab element and the intermediate elastic portion of the disposal tab, wherein the blanking film optionally consists of two blanking films portions. The blanking film portions are arranged between the intermediate elastic portion and the inelastic portions such that the contact area of the adhesive connecting these is minimized. Such a blanking film is advantageous because the elasticity of the intermediate elastic portion is maintained due to the smaller adhesive contact area between the intermediate elastic portion and the two inelastic portions. Using two blanking film portions has the advantage that the adhesive contact area is even further reduced, i. e. the elasticity of the intermediate elastic portion is maintained to a larger degree.

In still another embodiment, a blanking film is arranged on the adhesive of the first tab element at a position underneath of the intermediate elastic portion covering the intermediate elastic of the next layer if wound up. Such a blanking film is advantageous because an easy and reliable anti-adhesive means is provided thereby preventing the intermediate elastic portion from blocking with an adhesive present of the inelastic portions.

In one embodiment, the disposal tab comprises a third tab element. Before use - the second and third tab elements are connected to each other through a non-releasable connection and through a releasable connection by arranging the third tab element on the second tab element, whereby a z-fold configuration is formed. Before use - the third tab element is detachable from the second tab element thereby releasing the z-fold configuration, while the second and third tab elements are still connected through the non-releasable connection. Such a third tab element is beneficial because when releasing the z-fold configuration, more longitudinal extension is achieved with the third tab element, which may provide for a better wrap around the disposable article being in a disposal configuration. This gives further flexibility where to arrange the disposal tab on a disposable article, i. e. even further spaced from an edge thereof (i. e. not so close to the diaper edge) compared to single fold. It is to be noted that the dimensions, materials and other properties or components, which are mentioned for a disposal tab having just a first and a second tab element, also apply to such a disposal tab having a third tab element.

In some embodiments, the first, second and third tab elements including the two inelastic portions and the intermediate elastic portion therebetween as such are not folded, i. e. these are in a substantially flat configuration except for a folded end of the first, second or third tab element in order to expose an adhesive thereon for providing a non-releasable connection between the first and second tab elements and/or between the second and third tab elements. Such a substantially flat configuration has the advantage that the overall thickness of the disposal tab can be kept reasonably low which may have advantages during manufacture (web handling) and use (disposal tab being comparably flat on a diaper surface to which the disposal tab may be attached).

In another embodiment, the third tab element comprises an adhesive on its first major surface. A blanking film is provided on the adhesive of the third tab element of the disposal tab such that the blanking film (substantially/at least) covers the exposed intermediate elastic portion. An easy and reliable anti-adhesive means is provided thereby preventing the intermediate elastic portion from blocking with an adhesive present of the inelastic portions by the use of a blanking film.

In some embodiments, a fingerlift may be present in the adhesive of the second tab element (if a first and second tab element is present) or third tab element (if a first, second and third tab element is present). In other words, the fingerlift is present on the outermost tab element seen from the first tab element. Typically, the fingerlift is formed by a film non-releasably attached to the adhesive of the second or third tab element at one end opposite to the end where the non-releasable connection is formed between the first and second tab element or between the second and third tab element, respectively. Such a fingerlift allows for an easier grasping of the disposal tab upon use as the end comprising the fingerlift does not adhere to the respective surface and - in case of a film used to form the fingerlift - a thicker portion is formed which may further support easier grasping of the disposal tab. Alternatively, the fingerlift may be formed by an adhesion inhibiting coating on the adhesive or by strip coating of the adhesive on the second or third tab element leaving an end adhesive-free.

A suitable material for the backing of the first, second and/or third (if present) tab elements may include polymeric films, non-woven materials, a combination of a polymeric film and a non-woven layer. The backing is, for example, preferably selected from a group of materials comprising polypropylene, polyvinylchloride, polyethylene terephthalate, polyethylene, polyolefin copolymers or blends of polyolefins such as, for example, blend of polypropylene and LPDE (low density polyethylene), non-woven and foamed materials. The thickness of the backing is preferably between 25 µm and 500 µm, and more preferably between 100 µm and 400 µm. The basis weight of the backing may be 25 to 100 g/m², preferably 30 to 90 g/m². If a fibrous layer is used as backing, typically a nonwoven web of thermoplastic polymer fibers may be used. Suitable processes for making the nonwoven web include, but are not limited to, airlaying, spunbond, spunlace, bonded melt blown webs and bonded carded web formation processes. Spunbond nonwoven webs are made by extruding a molten thermoplastic as filaments from a series of fine die orifices in a spinneret. The diameter of the extruded filaments is rapidly reduced under tension by, for example, non-eductive or eductive fluid-drawing or other known spunbond mechanisms, such as described in US Patent Nos. 4 340653 (Appel et al.); 3 692 618 (Dorschner et al.); 3 338 992 and 3 341 394(Kinney); 3 276 944 (Levy); 3 502 538 (Peterson); 3 502 763 (Hartman); and 3 542 615 (Dobo et al.) The spunbond web is preferably bonded. The nonwoven web layer also may be made from bonded carded webs. Carded webs are made from separated staple fibers, which fibers are sent through a combing or carding unit which separates and aligns the staple fibers in the machine direction so as to form a generally machine direction-oriented fibrous nonwoven web. However, randomizers can be used to reduce this machine direction orientation. Once the carded web has been formed, it is then bonded by one or more of several bonding methods to give it suitable tensile properties. One bonding method is powder bonding wherein a powdered adhesive is distributed through the web and then activated, usually by heating the web and adhesive with hot air. Another bonding method is pattern bonding wherein heated calender rolls or ultrasonic bonding equipment are used to bond the fibers together, usually in a localized bond pattern though the web can be bonded across its entire surface if so desired. Generally, the more the fibers of a web are bonded together, the greater the nonwoven web tensile properties.

A suitable adhesive for use with the disposal tab according to the present disclosure may include pressure-sensitive adhesives including rubber-based adhesives (also called rubber-resin adhesives) which comprise natural or synthetic rubber materials and typically also tackifying resins in order to render the rubber materials tacky. Preferred examples of rubber-based pressure-sensitive adhesives are the polystyrene polyisoprene block copolymers tackified with synthetic polyterpene resins. Suitable pressure-sensitive adhesives furthermore include acrylate-based pressure sensitive adhesives such as, for example, those disclosed in US RE 24,906 or US 4,710,536. The adhesives mentioned above are given only by way of example, and the person skilled in the art can select other adhesives known in the art without any inventive effort. The thickness of the adhesive may be 10 to 200 µm, preferably about 20 to 100 µm.

A suitable elastic material for the intermediate elastic portion of the disposal tab according to the present disclosure may be include an elastic film comprising a material that exhibits elastomeric properties at ambient conditions, i. e. the material will substantially resume its original shape after being stretched. Preferably, the elastomer will sustain only a small permanent set following deformation and relaxation, which set is preferably less than 30 % and more preferably less than 20 % of the original 50 % to 500 % stretch. The elastomeric material can be either pure elastomers or blends with an elastomeric phase or content that will still exhibit substantial elastomeric properties at room temperature. Suitable elastomeric thermoplastic polymers include block copolymers or the like. These block copolymers are described in for example US 3,265,765; 3,562,356; 3,700,633; 4,116,917 and 4,156,673. Particularly useful are styrene/isoprene, butadiene or ethylenebutylene/styrene block copolymers. Generally, the block copolymers contain an A block and a B block. These blocks may be arranged in any order including linear, radial, branched or star block copolymers. Other useful elastomeric compositions can include elastomeric polyurethanes, ethylene copolymers such as ethylene vinyl acetates, ethylene/propylene copolymer elastomers or ethylene/propylene/diene terpolymer elastomers. Blends of these elastomers with each other or with modifying elastomers are also contemplated.

A suitable material for the fingerlift of the second or third tab element of the disposal tab according to the present disclosure may be a polymeric film including materials such as polypropylene, polyethylene, polyesters etc. having a basis weight of 10 to 500 g/m², preferably 30 to 90 g/m². and a thickness of 25 µm and 500 µm, and more preferably between 100 µm and 400 µm. The material for the fingerlift may be similar or the same as mentioned above for the backing of the first, second or - if present - third tab element. The extension of the fingerlift in machine direction is essentially the same as the extension of the disposal tab in machine direction. The extension of the disposal tab in cross-direction is typically 3 to 10 mm.

A suitable material for the blanking film of the disposal tab according to the present disclosure may be similar or the same material as mentioned above for the backing of the first, second or - if present - third tab element.

A suitable anti-adhesive means for the use with the disposal tab according to the present disclosure may include a low adhesion backside comprising an anti-adhesive coating, preferably a silicone coating. Other anti-adhesive means include an anti-adhesive paper, anti-adhesive film, a cover strip or a liner.

A disposal tape according to the present disclosure may - for example - be made by a method comprising the steps of providing a first tab element web, providing a second tab element web comprising a first and second inelastic portion web connected by an intermediate elastic portion web therebetween, e. g. by unwinding such a web from a roll. Optionally, the second tab element web is made by providing a first and second inelastic portion web, providing an intermediate elastic portion web, connecting the first and second end portion web to the intermediate elastic portion web, e. g. with an adhesive, such that the intermediate elastic portion web is arranged between the first and second inelastic portion web. The method may further comprise the step of joining the first and second tab element such that one end of the first tab element web is folded onto itself to expose an adhesive present thereon contacting an adhesive on the second tab element web to form a non-releasable connection therebetween. The second tab element web may then be arranged onto the first tab element web such that an adhesive of the second tab element web is in contact with a low adhesion backside of the first tab element web. A releasable connection between the first and second tab element web is formed thereby. The method may also comprise the step of winding the obtained disposal tape into a roll for storage and/or further handling. The method may also optionally comprise the step of arranging a third tab element web onto the second tab element web by folding an end of the second tab element web such that an adhesive is exposed. The exposed adhesive may then be connected to an adhesive present on the third tab element web such that a non-releasable connection is formed between the second and third tab element web. The third tab element web may then be arranged onto the second tab element web such that an adhesive of the third tab element web is in contact with a low adhesion backside of the second tab element web. A releasable connection between the second and third tab element web is formed thereby. In the above-mentioned method, the position of the second and third tab element may be exchanged with each other, i. e. the third tab element may be connected to the second tab element on the one hand and to the first tab element on the other hand.

The invention was described in various embodiments above. It is understood by a person skilled in the art, that one, several or all of the above-mentioned embodiments can be combined with each other.

The invention will now be described in more detail with reference to the following Figures exemplifying particular embodiments of the invention:
- Fig. 1: is a schematic side view of a disposal tab according to an embodiment of the present disclosure.
- Fig. 2: is a schematic side view of a disposal tab according to another embodiment of the present disclosure.
- Fig. 3: is a schematic side view of a disposal tab according to a further embodiment of the present disclosure.
- Fig. 4: is a schematic side view of a disposal tab according to yet a further embodiment of the present disclosure.
- Fig. 5: is a schematic side view of a disposal tab according to yet another embodiment of the present disclosure.
- Fig. 6: is a schematic side view of a disposal tab according to a further embodiment of the present disclosure.
- Fig. 7a: is a schematic side view of a disposal tab according to another embodiment of the present disclosure.
- Fig. 7b: is a schematic side view of a disposal tab according to another embodiment of the present disclosure.
- Fig. 8: is a schematic side view of a disposal tab according to yet a further embodiment of the present disclosure.
- Fig. 9: is a schematic side view of a disposal tab according to yet another embodiment of the present disclosure.
- Fig. 10: is a schematic side view of a disposal tab according to a further embodiment of the present disclosure.
- Fig. 11: is a schematic side view of a disposal tab according to another embodiment of the present disclosure.
- Fig. 12: is a perspective schematic view of a pant or short style diaper with a disposal tab according to the present disclosure.
- Fig. 13: is a perspective schematic view of a pant or short style diaper with a disposal tab according to the present disclosure used to compact the diaper after use.
- Fig. 14: is a cross-sectional schematic view of the disposal tab of Fig. 6 attached to the outer surface of a disposable article.
- Fig. 15: is a cross-sectional schematic view of the disposal tab of Fig. 7 attached to the outer surface of a diaper.
- Fig. 16: is a perspective schematic view of a disposal tape according to the present disclosure comprising a plurality of disposal tabs according to the present disclosure in an endless form as well as of a disposal tab according to the present disclosure cut therefrom.
- Fig. 17: is a schematic side view of a disposal tab according to yet a further embodiment of the present disclosure.

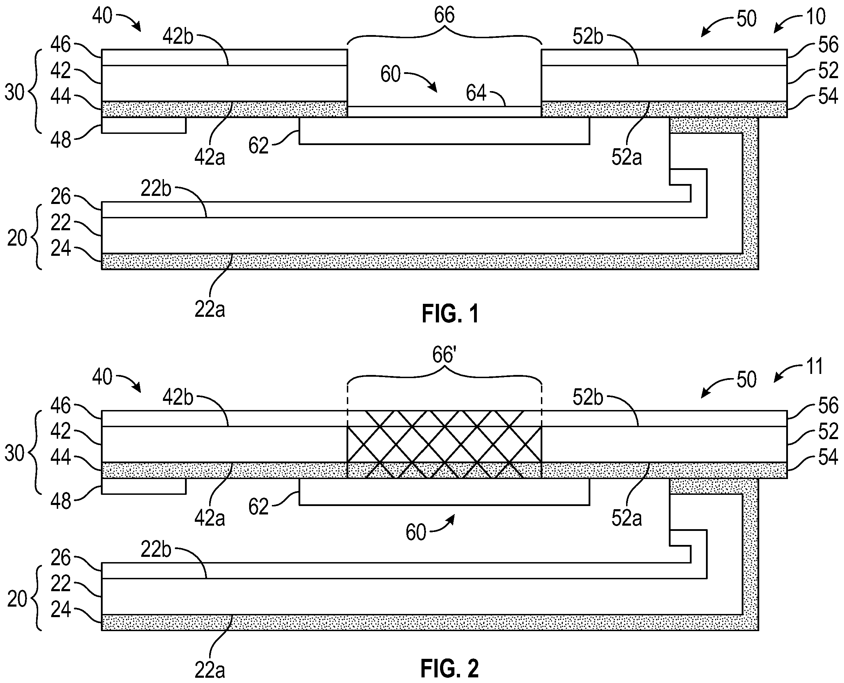

In some of the Figs., although the first, second or - if present - third tab elements and/or other parts such as blanking films, fingerlifts, center strips, intermediate elastic portions, are shown slightly spaced from the first tab element 20 in Fig. 1, it is meant, in particular when shown vis-a-vis to an adhesive, that these contact each other.

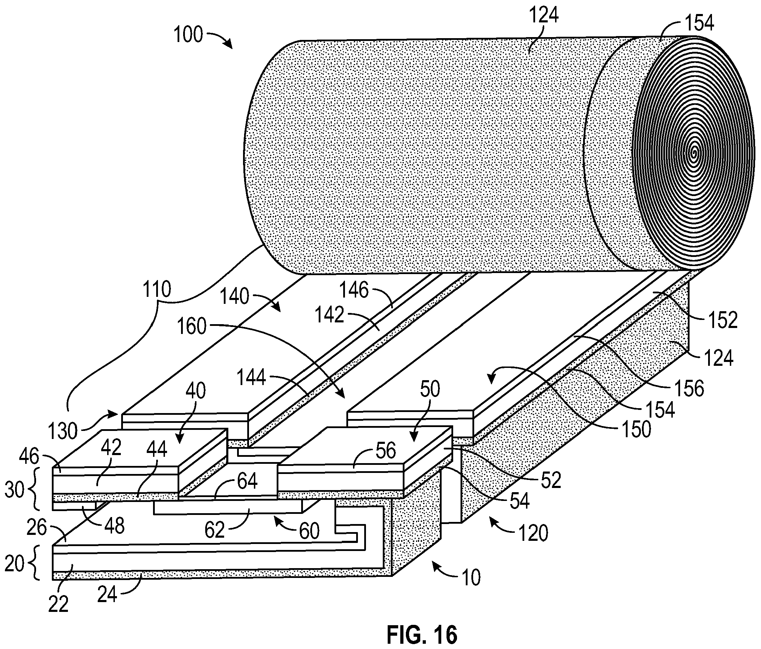

Fig. 1 shows a first embodiment of the disposal tab 10 according to the present disclosure. The disposal tab 10 comprises a first and second tab element 20, 30 each having a backing 22, 42, 52 with a first and second major surface 22a, 22b, 42a, 42b, 52a, 52b. The first and second tab elements 20, 30 comprise an adhesive 24, 44, 54 on one of their major surfaces 22a, 42a, 52a. The first tab element 20 further comprises on its second major surface 22b opposite to the major surface 22a with the adhesive 24 thereon a low adhesion backside 26 to allow the adhesives 44, 54 to be released therefrom, thereby forming a releasable connection between the first and second tab element 20, 30. As mentioned above, the adhesive 54 of one of the inelastic portions 50 is also non-releasable attached to the exposed adhesive 24 on the second major surface 22b of the first tab element 20 for a non-releasable connection. Prior to use, the adhesives 44, 54 contact the low adhesion backside 26 of the first tab element 20 thereby forming a releasable connection between the first and second tab element 20, 30. The adhesive 24 of the first tab element 20 is intended to attach the disposal tab 10 to the outer surface of a disposable article (not shown in Fig. 1). The first tab element 20 is folded on one end onto itself such that the adhesive 24 is exposed to the other side. The adhesive 24 is thereby in contact with the adhesive 54 of the second tab element 30 forming a non-releasable connection between the first and second tab elements 20, 30. The second tab element 30 comprises two inelastic portions 40, 50 each having a backing 42, 52 with a first and second major surface 42a, 42b, 52a, 52b, whereby the intermediate elastic portion 60 is non-releasably attached to the first major surfaces 42a, 52a of inelastic portions 40, 50 by the adhesives 44, 54 to connect the inelastic portions 40, 50 and the intermediate elastic portion 60. The second tab element 30 and the two inelastic portions 40, 50, respectively, further comprise a low-adhesion backside 46, 56 on one major surface 42b, 42a opposite to the major surfaces 42a, 52a having the adhesives 44, 54 thereon. An exposed area 66 is present on the intermediate elastic portion 60 between the two inelastic portions 40, 50. The exposed area 66 is covered with a low adhesion backside 64 allowing an adhesive to be released therefrom. Such an adhesive may, for example, contact the low adhesive backside 64 when winding up a tape comprising a plurality of disposal tabs 10 into a roll (not shown, see Fig. 16), whereby one layer on such a roll may contact the layer below and above, respectively. The second tab element 30 further comprises a fingerlift 48 on the inelastic portion 40. The fingerlift 48 consists of a blanking film attached to the adhesive 44 of the second tab element 30 at a free end thereof and helps a user of the disposal tab 10 to grasp and lift the disposal tab 10 upon use. The second tab element 30 further comprises an extension formed by the second tab element 30 and protruding beyond the first tab element 20 on the free end of the second tab element 30 opposite to the fingerlift 48. The extension provides for an exposed portion of the adhesive 54 of the second tab element 30. The extension may be bonded directly to the outer surface of a disposable article to which the disposal tab 10 may be attached (not shown here).

Fig. 2 shows a different embodiment of the disposal tab 11 according to the present disclosure. The disposal tab 11 is similar to the disposal tab 10 as shown in Fig. 1 except that there is no exposed area 66 present on the intermediate elastic portion 60 between the two inelastic portions 40, 50 here. Instead, Fig. 2 shows a covered portion 66' between the two inelastic portions 40, 50 of the second tab element 30. The covered portion 66' covers the portion of the intermediate elastic portion 60 which would otherwise be exposed. The covered portion 66' is formed by the two backings 42, 52 of the two inelastic portions 40, 50. Identical to the first and second inelastic portions 40, 50, the covered portion 66' also has a backing and an adhesive on one of its major surface with which the covered portion is attached onto the intermediate elastic portion 60. The covered portion 66' further comprises a low adhesion backside on the major surface opposite to the one bearing the adhesive such that an adhesive coming in contact therewith can easily be released therefrom without any damage. The covered portion 66' comprises slits or cuts therein allowing elongation of the covered portion 66' in order to maintain the elasticity of the underlying intermediate elastic portion 60.

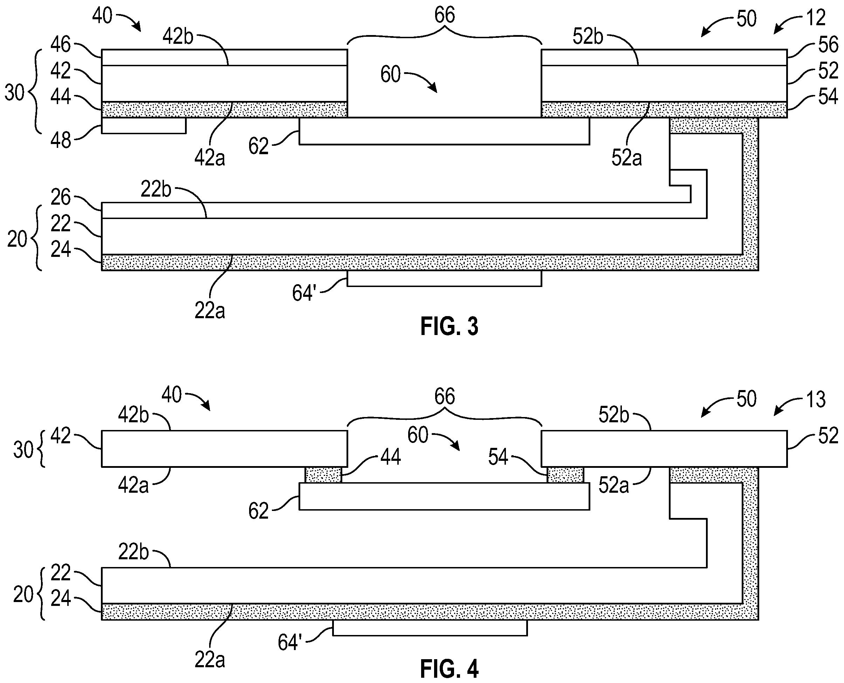

Fig. 3 shows a different embodiment of the disposal tab 12 according to the present disclosure. The disposal tab 12 is similar to the disposal tab 10 as shown in Fig. 1 except that there is no low adhesion backside 64 arranged on one of the major surfaces of the intermediate elastic portion 60. Instead, a blanking film 64' is attached to the adhesive 24 of the first tab element 20. When winding up a disposal tape comprising a plurality of disposal tabs 12 into a roll (not shown, see Fig. 16), the blanking film 64' covering the adhesive 24 prevents the intermediate elastic portion 60 from coming into contact with the adhesive 24 of the disposal tape layer above and from blocking when again unwinding the disposal tape.

Fig. 4 shows a different embodiment of the disposal tab 13 according to the present disclosure. The disposal tab 13 is similar to the disposal tab 12 as shown in Fig. 3 except that there is no low adhesion backside arranged on the first tab element 20 and that-the extension of the adhesive layers 44, 54 of the two inelastic portions 40, 50 is different. That is, the adhesives 44, 54 only extend in the contact area between the inelastic portions 40, 50 and the intermediate elastic portion 60 such that there is no exposed adhesive in the major surfaces 42a, 52a of the two inelastic portions 40, 50. Therefore, no low adhesion backside is required like in Fig. 3 to protect the second tab element 30 from blocking with an adhesive on the first tab element 20. In this embodiment, the releasable connection between the first and second tab elements 20, 30 is formed in a different way compared to the previous Figs. Here, an additional releasable hold down means, e. g. a releasable ultrasonic bonding dot or a small layer of hotmelt adhesive (both not shown in Fig. 3) may provide for the releasable connection between the first and second tab elements 20, 30. As there is no adhesive present at the free end of the first inelastic portion 40, there is no fingerlift arranged and required, respectively, in order to ease grasping and lifting of the disposal tab 10 upon use. Furthermore, as there is adhesive only in the area between the first and second tab elements 20, 30, i. e. no exposed adhesive present at the second inelastic portion 50, there is no bonding provided to a disposable article in that area like it was with the embodiment of Figs. 1 to 3. Therefore, the extension of the second inelastic portion 50 protruding beyond the first tab element 20 may be omitted here. The non-releasable connection between the first and second tab elements 20, 30 is provided - different to Figs. 1 to 3 - solely by the adhesive 24 of the first tab element being exposed by folding one end of the first tab element 20 onto itself as shown in Figs. 1 to 3 and contacting the first major surface 52a of the second tab element 30, which has in this case no adhesive thereon.

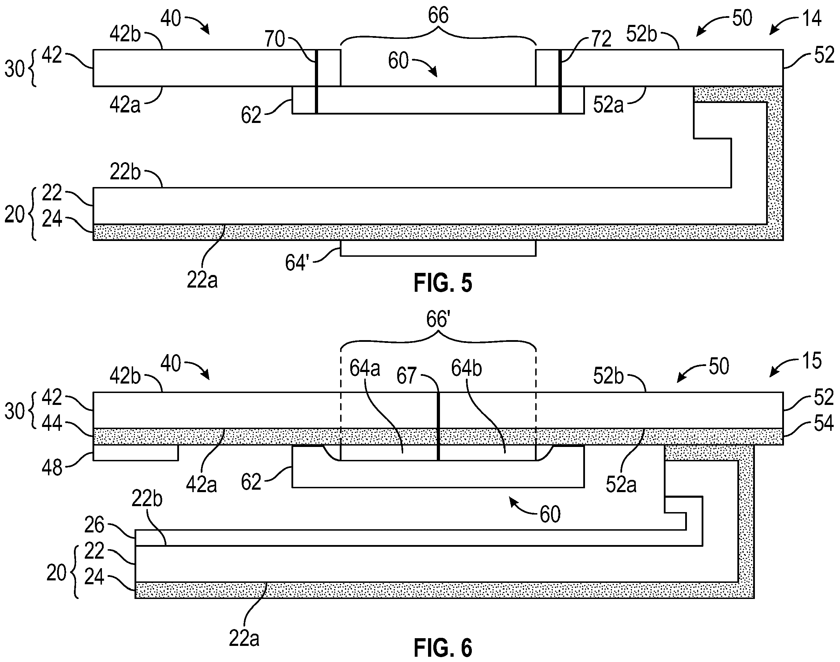

Fig. 5 shows a different embodiment of the disposal tab 14 according to the present disclosure. The disposal tab 14 is similar to the disposal tab 13 as shown in Fig. 4 except that there is no adhesive at all present on the major surfaces 42a, 52a of the first and second inelastic portions 40, 50 of the second tab element 30. The connection 70, 72 between the first and second inelastic portions 40, 50 to the intermediate elastic portion 60 is provided by an ultrasonic bond. Similar to the embodiment is shown in Fig. 4, an additional hold down means (not shown in Fig. 5) for providing the releasable connection between the first and second tab elements 20, 30 may be arranged as described under Fig. 4. As there is no adhesive present at the free end of the first inelastic portion 40, there is no fingerlift arranged and required, respectively, in order to ease grasping and lifting of the disposal tab 10 upon use. Furthermore, an extension of the second inelastic portion 50 - as shown for the embodiments for Figs. 1 to 3 - protruding beyond the first tab element 20 was omitted here. The non-releasable connection between the first and second tab elements 20, 30 is provided - similar to Fig. 4 - solely by the adhesive 24 of the first tab element being exposed by folding one end of the first tab element 20 onto itself as shown in Figs. 1 to 3 and contacting the first major surface 52a of the second tab element 30, which has in this case no adhesive thereon.

Fig. 6 shows a different embodiment of the disposal tab 15 according to the present disclosure. The disposal tab 15 is similar to the disposal tab 11 as shown in Fig. 2 except that the covered portion 66' is formed in a different way. Here, the covered portion 66' consists of extensions of the backings 42, 52 of the first and second inelastic portions 40, 50 such that these abut each other indicated with reference sign 67. Alternatively, the covered portion 66' may be formed by one backing for the first and second inelastic portions 40, 50 having a slit in the middle (indicated at 67). The blanking film portions 64a, 64b are arranged between the adhesives 42, 52 of the first and second inelastic portion 40, 50 and the intermediate elastic end portion 60 thereby preventing contact between the adhesives 42, 52 and the intermediate elastic portion 60 in that area allowing elongation of the covered portion 66' and thereby maintaining the elasticity of the intermediate elastic portion 60.

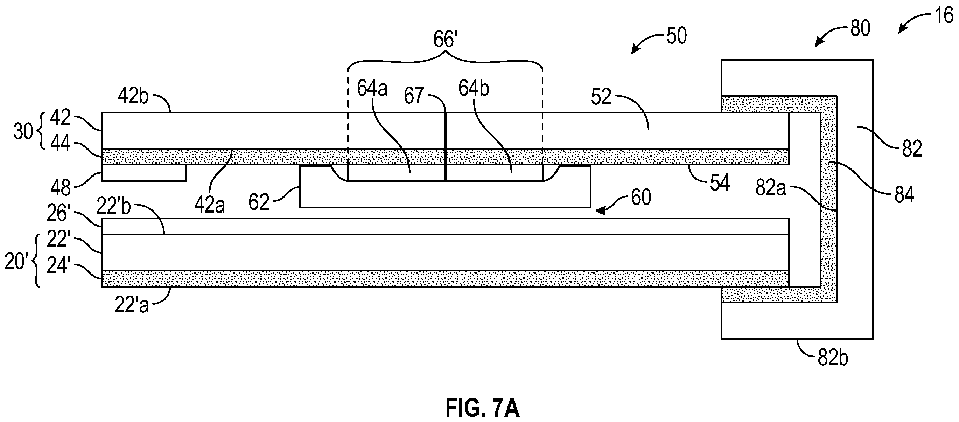

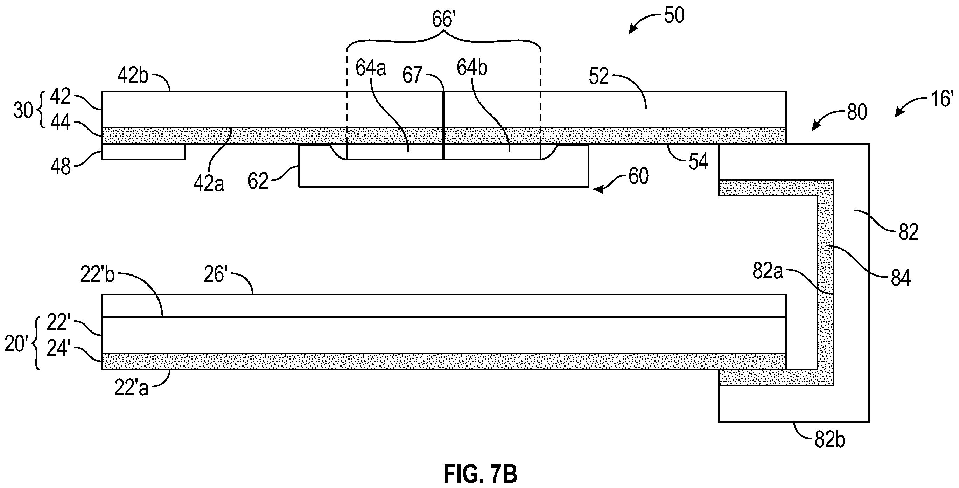

Fig. 7a shows a different embodiment of the disposal tab 15' according to the present disclosure. The disposal tab 15' is similar to the disposal tab 15 as shown in Fig. 6 except that there is non-releasable connection between the first and second tab element 20, 30 is formed in a different way. The first element 20' of the disposal tab 15' of Fig. 7 does not exhibit a folded end, but has a c-shaped center strip 80 with folded ends which embrace or encompass one end of each of the first and second tab elements 20', 30. The center strip 80 has a backing 82 and an adhesive 84 on one of its major surfaces 82a facing towards the first and second tab elements 20', 30, whereby the center strip 80 is non-releasably connected to the first and second tab elements 20', 30 thereby forming the non-releasable connection between the first and second tab elements 20', 30. The second major surface 82b of the center strip 80 is opposite to the first major surface 82a.

Fig. 7b shows a different embodiment of the disposal tab 16' according to the present disclosure. The disposal tab 16' is similar to the disposal tab 16 as shown in Fig. 7a except that the center strip 80' is arranged in a different way compared to the center strip 80 in Fig. 7a. Here, the center strip 80' only embraces or encompasses with its folded ends - different to Fig. 7a - only one end of the first tab element 20', whereby the center strip 80' contacts the adhesive 54 of the second inelastic portion 50 of the second tab element 30. Thereby, the non-releasable connection between the first and second tab elements 20, 30 is provided. As shown, the center strip 80' comprises a backing 82 having a first and second major surface 82a, 82b, only one of which comprises an adhesive 84. As an alternative, the center strip 80' may not have an adhesive on one of its majors surfaces 82a, 82b at all as the center strip 80' connects to the adhesive 22'a of the first tab element 20' on the one hand and to the adhesive 54 of the second tab element 30 on the other hand (different to the embodiment of Fig. 6, where the center strip connects to the second major surface 52b of the second tab element 50 without an adhesive present thereon).

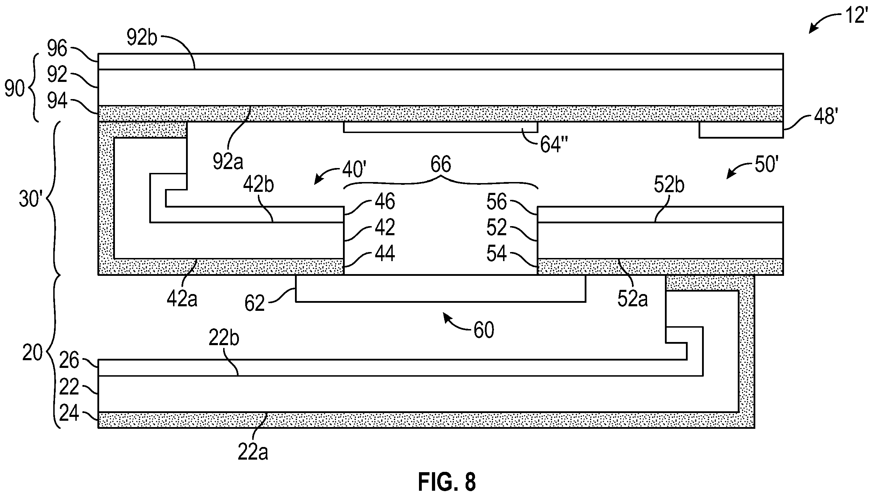

Fig. 8 shows a different embodiment of the disposal tab 12' according to the present disclosure. The disposal tab 12' is similar to the disposal tab 10 as shown in Fig. 1 except that there is an additional third tab element 90 present. The third tab element 90 comprises a backing 92 with a first and second major surface 92a, 92b. An adhesive 94 is arranged on the first major surface 92a and a low adhesion backside is arranged on the second major surface 92b of the third tab element 90. The second tab element 30 is folded on one end onto itself such that the adhesive 44 is exposed to the other side. The adhesive 44 is in non-releasable contact with the adhesive 94 of the third tab element 90 thereby forming a non-releasable connection between the second and third tab elements 30, 90. The third tab element 90 further comprises a blanking film 64" covering the adhesive 94 in an area which would otherwise come in contact to the intermediate elastic portion 60 of the second tab element 30. Thereby, the adhesive 94 of the third tab portion 90 does not adhere to the intermediate elastic portion 60. The first and second inelastic portions 40, 50 of the second tab element 30 comprise each a low adhesion backside 46, 56 on their second major surfaces 42b, 52b allowing the adhesive 94 to be released therefrom. Thereby, a releasable connection is formed between the second and third tab element 30, 90. The releasable connection and the non-releasable connections between the tab elements are shown and described above under Fig. 1. The third tab element 90 further comprises a fingerlift 48". In the embodiment shown in Fig. 8, the fingerlift 48" consists of a blanking film 48" attached to the adhesive 94 of the third tab element 90 at a free end thereof and helps a user of the disposal tab 12' to grasp and lift the disposal tab 12' upon use.

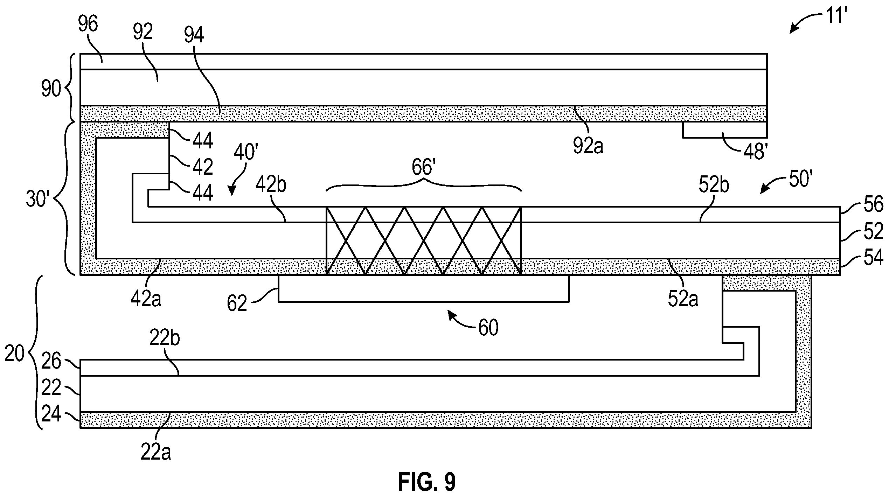

Fig. 9 shows a different embodiment of the disposal tab 11' according to the present disclosure. The disposal tab 11' is similar to the disposal tab 16 as shown in Fig. 8 except that there is no exposed area 66 present on the intermediate elastic portion 60 between the two inelastic portions 40, 50 here. Instead, Fig. 9 shows a covered portion 66' between the two inelastic portions 40, 50. The covered portion 66' covers the portion of the intermediate elastic portion 60 which would otherwise be exposed between the first and second inelastic portions 40, 50 of the second tab element 30 (similar to as shown in Fig. 2). The covered portion 66' is formed by the two backings 42, 52 of the two inelastic portions 40, 50. Identical to the first and second inelastic portions 40, 50, the covered portion 66' also has a backing and an adhesive on one of its major surface with which the covered portion is attached onto the intermediate elastic portion 60. The covered portion 66' further comprises a low adhesion backside on the major surface opposite to the one bearing the adhesive such that an adhesive coming in contact therewith can easily be released without any damage. The covered portion 66' comprises slits or cuts therein allowing elongation of the covered portion 66' in order to maintain the elasticity of the underlying intermediate elastic portion 60.

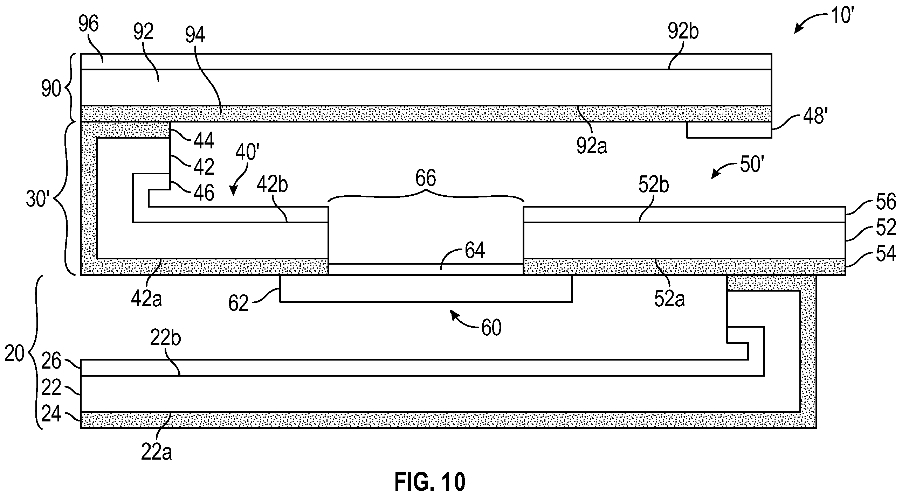

Fig. 10 shows a different embodiment of the disposal tab 10' according to the present disclosure. The disposal tab 10' is similar to the disposal tab 12' as shown in Fig. 8 except that there is no blanking film 64" arranged on the adhesive 94 of the third tab element. Instead, the exposed area 66 is covered with a low adhesion backside 64 allowing an adhesive to be released therefrom similar to as shown in Fig. 1. Such an adhesive may be the adhesive 94 of the third tab element 90 releasably attached to the second tab element 30.

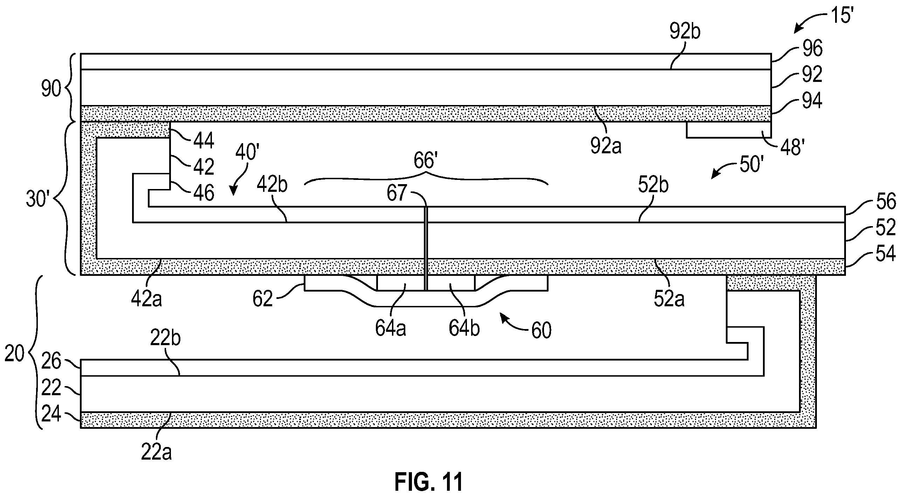

Fig. 11 shows a different embodiment of the disposal tab 15' according to the present disclosure. The disposal tab 15' is similar to the disposal tab 12' as shown in Fig. 8 except that there is no exposed area 66 present on the intermediate elastic portion 60 between the two inelastic portions 40, 50 here. Instead, Fig. 11 shows a covered portion 66' between the two inelastic portions 40, 50 of the second tab element 30. The covered portion 66' covers the portion of the intermediate elastic portion 60 which would otherwise be exposed between the first and second inelastic portions 40, 50 of the second tab element 30. The covered portion 66' consists of extensions of the backings 42, 52 of the first and second inelastic portions 40, 50 such that these abut each other indicated with reference sign 67. The blanking film portions 64a, 64b are arranged between the adhesives 42, 52 of the first and second inelastic portions 40, 50 and the intermediate elastic end portion 60 thereby preventing contact between the adhesives 42, 52 and the intermediate elastic portion 60 in that area allowing the covered portion 66' to elongate thereby maintaining the intermediate elastic portion 60 elastic. The covered portion 66' also comprises a low adhesion backside on the major surface opposite to the one bearing the adhesive such that an adhesive coming in contact therewith can easily be released without any damage. Such an adhesive maybe the adhesive 94 of the third tab portion 90 being releasable in contact to the second tab portion 30.

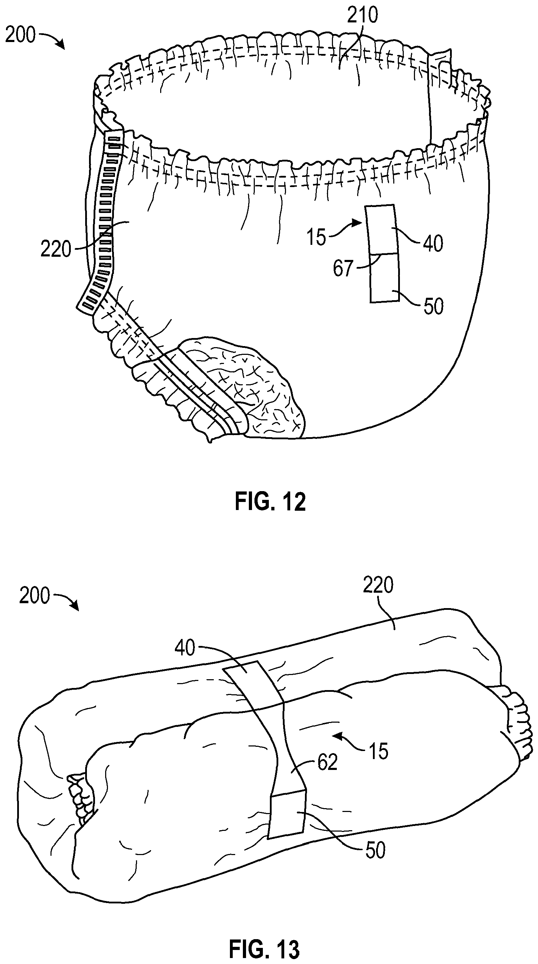

The disposal tape tab 15 of the present disclosure can be particularly advantageously used in connection with pants or shorts style infant diapers or adult incontinence articles 200 as schematically illustrated in Fig. 12. As shown, the disposal tab 15 is attached to the outer surface 220 of the diaper in a rear waist region thereof. The two inelastic portions 40, 50 abut each other indicated at 67 prior to use of the disposal tab 15. After use of such a pant style diaper 200, the pant is typically torn apart along at least one of its seams prior to rolling it up. Removing the soiled diaper over the legs would typically contaminate the legs.

Fig. 13 shows a used (soiled) diaper 200 in a rolled-up state after it has been torn along the seams. The disposal tape tab 15 has been elongated thereby stretching the (elastic) backing 62 of the intermediate elastic portion 60 of the disposal tab 15 and moving the two inelastic portions 40, 50 away from each other. The disposal tab 15 is used to hold the soiled diaper in a compacted or disposal configuration which typically is a rolled-up configuration as shown in Fig. 13. Upon use of the disposal tab 15, the first end portion 40 was - as mentioned above - moved away from the second end portion 50 thereby elongating the disposal tab 15, whereby the first end portion 40 has been non-releasably attached to the outer surface 220 of the diaper 200 such that the rolled-up configuration is maintained.

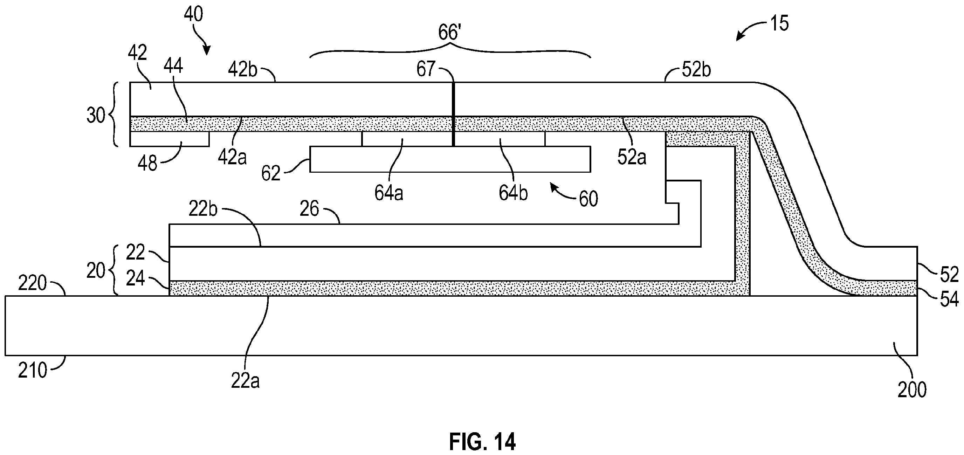

Fig. 14 shows the disposal tab 15 according to the embodiment of Fig. 6 being attached to the outer surface 220 of a disposable article 200. The disposal tab 15 is attached with the adhesive 24 of the first tab element 20 on the one hand and with the adhesive 54 of the extension of the second tab element 30 on the other hand to the outer surface 220. As shown in Figs. 1 to 3, the extension of the second tab element 30 provides for an exposed portion of the adhesive 54 of the second tab element 30. The extension may be bonded directly to the outer surface 220 of a disposable article 200 to which the disposal tab 15 is attached (not shown here) Although the first and second tab elements 20, 30 are shown in a slightly spaced configuration, it is meant that these are in contact with each other prior to use. Also, the backing 62 of the intermediate elastic portion 60 is shown spaced from the adhesives 44, 54, it is meant that the backing 62 and the adhesives 44, 54 are in contact with each other. Furthermore, the inner surface 210 of the disposable article 200 is shown in Fig. 14 as well.

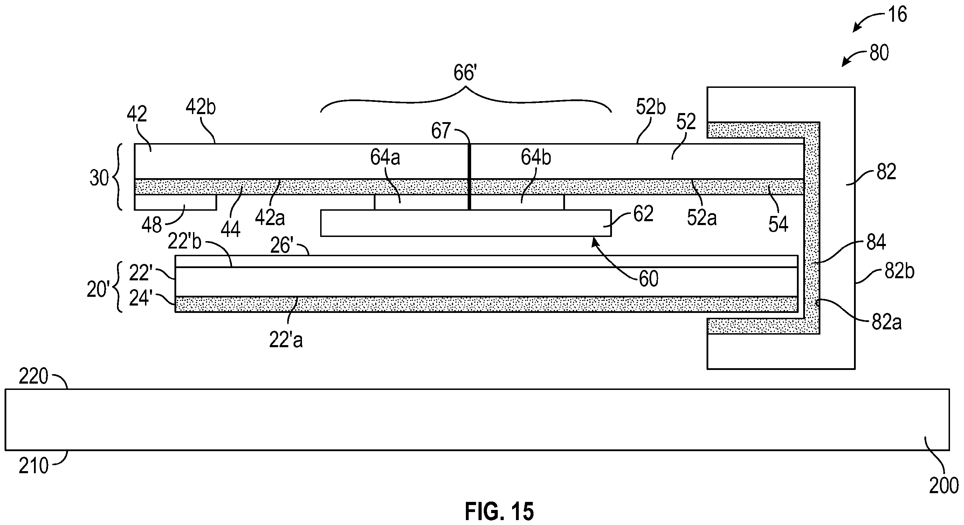

Fig. 15 shows the disposal tab 16 according to the embodiment of Fig. 7 being attached to the outer surface 220 of a disposable article 200. The disposal tab 16 is attached with the adhesive 24 of the first tab element 20 to the outer surface 220. Although the first and second tab elements 20, 30 are shown in a slightly spaced configuration, it is meant that these are in contact with each other prior to use. Also, the backing 62 of the intermediate elastic portion 60 is shown spaced from the adhesives 44, 54, it is meant that the backing 62 and the adhesives 44, 54 are in contact with each other. Furthermore, the inner surface 210 of the disposable article 200 is shown in Fig. 15 as well.

Fig. 16 illustrates a roll 100 of the disposal tape 110 comprising a plurality of disposal tabs 10 in an endless form. The disposal tab 10 is illustrated and described in more detail in Fig. 1. Individual disposal tabs 10 can be cut from the disposal tape 110 after unwinding it from the roll 100 and thereby defining a main longitudinal direction or machine direction and applying a cut in cross-direction, i. e. the cut is applied in a direction transverse to the main longitudinal direction of the disposal tape 110 (machine direction). The disposal tape 110 comprises a first and second tab element web 120, 130, only parts of which are visible in Fig. 16. The first tab element web 120 comprises a backing with a low adhesion backside on one of its major surfaces (both not visible) and an adhesive 124 on the opposite major surface. The second tab element web 130 comprises a first and second inelastic portion web 140, 150 connected by an intermediate elastic portion web 160. The first and second inelastic portion web 140, 150 comprises an adhesive 144, 154 on one of their major surface connecting to the intermediate elastic portion web 160 and on the other major surfaces a low adhesion backside 146, 156 to allow the adhesive 124 to be released therefrom when winding up and later unwinding the disposal tape 110 into and from a roll 100. The latter allows for easy unwinding of such a disposal tape 110 from the roll 100 without blocking. The intermediate elastic portion web 160 of the disposal tape 110 comprises between the first and second inelastic portion webs 140, 150 an exposed area of the intermediate elastic portion web 160 having a low adhesion backside arranged thereon, which has been omitted for simplification in Fig. 16. Furthermore, Fig. 16 shows a disposal tab 10 as described in Fig. 1, which was cut from the disposal tape 110.

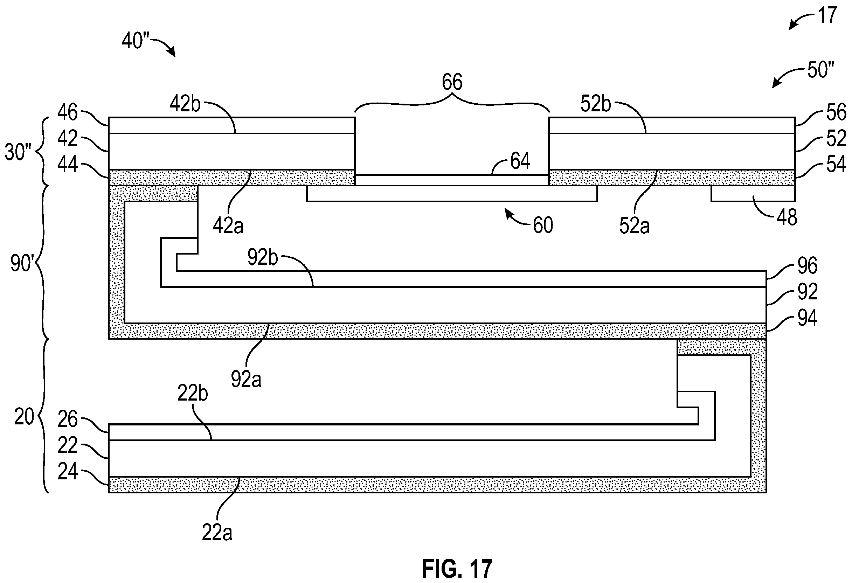

Fig. 17 shows a different embodiment of the disposal tab 17 according to the present disclosure. The disposal tab 17 is similar to the disposal tab 12' as shown in Fig. 8 except that the positions of the second and third tab elements 30", 90' are different compared to the position of the second and third tab elements 30', 90 in Fig. 8. Here, the third tab element 90' is arranged on the first tab element 20, whereas the second tab element 30" is arranged on the third tab element 90'. The third tab element 90 comprises a backing 92 with a first and second major surface 92a, 92b. An adhesive 94 is arranged on the first major surface 92a and a low adhesion backside is arranged on the second major surface 92b of the third tab element 90. The third tab element 90' is folded on one end onto itself such that the adhesive 94 is exposed to the other side. The adhesive 94 is in non-releasable contact with the adhesive 44 of the second tab element 30" thereby forming a non-releasable connection between the second and third tab elements 30", 90'. The first and second inelastic portions 40", 50" of the second tab element 30" comprise each a low adhesion backside 46, 56 on their second major surfaces 42b, 52b allowing an adhesive to be released therefrom which may come into contact with the second tab element 30" when winding up a disposal tape comprising a plurality of disposal tabs 17 into a roll, i. e. an adhesive from a layer above may contact with its adhesive of the first tab element 20 the second tab element 30" of the layer below. The third tab element 90' comprise each a low adhesion backside 96 on its second major surfaces 92b allowing the adhesive 44, 54 to be released therefrom Thereby, a releasable connection is formed between the second and third tab element 30, 90. The first tab element 20 comprises a low adhesion backside 26 on its second major surface 22b, whereby a releasable connection between the first and third tab element 20, 90' is provided by arranging the third tab element 90' with its adhesive 94 on the low adhesion backside 26 of the first tab element 20. The second tab element 30" further comprises a fingerlift 48". In the embodiment shown in Fig. 8, the fingerlift 48" consists of a blanking film 48" attached to the adhesive 54 of the second tab element 30" at a free end thereof and helps a user of the disposal tab 17 to grasp and lift the disposal tab 17 upon use.

## Claims

1. A disposal tab (10, 10', 11, 11', 12, 12', 13, 14, 15, 15', 16, 16') for securing a disposable article (200) in a disposal configuration, the tab (10, 10', 11, 11', 12, 12', 13, 14, 15, 15', 16, 16') comprises:
a. a first tab element (20, 20') for attaching the tab (10, 10', 11, 11', 12, 12', 13, 14, 15, 15', 16, 16') onto a disposable article (200),
b. a second tab element (30, 30') configured and adapted to elongate the tab (10, 10', 11, 11', 12, 12', 13, 14, 15, 15', 16, 16') along its major longitudinal extension, comprising two inelastic portions (40, 50) and an intermediate elastic portion (60) therebetween and
wherein the first and second tab elements (20, 20', 30, 30') are connected to each other through a non-releasable connection (24, 54, 82, 84) and - before use - through a releasable connection (24, 24') by arranging the second tab element (30, 30') on the first tab element (20, 20'), whereby a folded configuration is formed,
wherein the non-releasable connection (24, 54, 82, 84) is formed either by one end of the first or second tab element (20, 20', 30, 30') being folded onto itself and attached to the other tab element (20, 20', 30, 30') or
by a c-shaped center strip (80) encompassing an end of at least the first and second tab element (20, 20', 30, 30'),
wherein - before use - the second tab element (30, 30') is detachable from the first tab element(20, 20') upon use of the tab (10, 10', 11, 11', 12, 12', 13, 14, 15, 15', 16, 16') thereby releasing the folded configuration, while the tab elements (20, 20', 30, 30') are still connected through the non-releasable connection (24, 54, 82, 84).

2. The disposal tab (10, 10', 11, 11', 12, 12', 13, 14, 15, 15', 16, 16') according to claim 1, wherein the non-releasable connection (24, 54, 82, 84) is made by means of adhesive bonding (24, 24'), thermobonding, ultrasonic bonding and/or cold pressure bonding and/or wherein the releasable connection is made by means of adhesive bonding, thermobonding, ultrasonic bonding and/or cold pressure bonding.

3. The disposal tab (10, 10', 11, 11', 12, 12', 13, 14, 15, 15', 16, 16') according to any one of the preceding claims, wherein the first and second tab element (20, 20', 30, 30') comprises an adhesive (24, 44, 54) on one of their major surface (22a, 22b, 42a, 42b, 52a, 52b).

4. The disposal tab (10, 10', 11, 11', 12, 12', 13, 14, 15, 15', 16, 16') according to any one of the preceding claims, wherein an end of the first tab element (20, 20') is folded onto itself and attached to the second tab element (30, 30') such that an extension of the second tab element (30, 30') is formed which protrudes beyond the first tab element (20, 20') and which is attachable to the disposable article (200).

5. The disposal tab (10, 10', 11, 11', 12, 12', 13, 14) according to any one of the preceding claims, wherein the ends of the two inelastic portions (40, 50) of the second tab element (30, 30') are spaced apart from each other.

6. The disposal tab (15, 15', 16, 16') according to any one of claims 1 to 4, wherein the ends of the two inelastic portions (40, 50) of the second tab element (30, 30') abut each other.

7. The disposal tab (10, 10', 11, 11', 12, 12', 13, 14, 15, 15', 16, 16') according to any one of the preceding claims, wherein the intermediate elastic portion (60) of the second tab element (30, 30') is connected to each of the two inelastic portions (40, 50) by means of adhesive bonding (44), thermobonding, ultrasonic bonding and/or cold pressure bonding.

8. The disposal tab (11, 11', 15, 16, 16') according to any one of the preceding claims, wherein a cover portion 66' comprising cuts therein is arranged on the major surface of the intermediate elastic portion (60) of the second tab element (30, 30') facing the adhesive of the second tab element (30, 30'), wherein the cover layer (66') optionally is formed by the backing (42, 52) of the second tab element (30, 30').

9. The disposal tab (15, 15', 16) according to any one of the claims 3 to 8, wherein the adhesives 44, 54 are provided on the first major surface (42a, 42b) of the second tab element (30) and wherein a blanking film (64) is arranged between the adhesive (44, 54) of the second tab element (30, 30') and the intermediate elastic portion (60), wherein the blanking film (64) optionally consists of two blanking films portions (64a, 64b).

10. The disposal tab (10', 11', 12', 15') according to any one of the preceding claims, further comprising a third tab element (90), wherein - before use - the second and third tab elements (30, 30', 90) are connected to each other through a non-releasable connection and through a releasable connection by arranging the third tab element on the second tab element, whereby a z-fold configuration is formed,
wherein - before use - the third tab element (90) is detachable from the second tab element (30, 30') thereby releasing the z-fold configuration, while the second and third tab elements (30, 30', 90) are still connected through the non-releasable connection (44, 94).

11. The disposal tab (10, 10') according to claim 10, wherein the third tab element (90) comprises an adhesive (94) on its first major surface (92a) and wherein a blanking film (64") is provided on the adhesive (94) of the third tab portion (90) such that the blanking film (64") (substantially/at least) covers the exposed intermediate elastic portion (60).

12. A disposal tape (110) from which disposal tabs (10, 10', 11, 11', 12, 12', 13, 14, 15, 15', 16, 16') according to any one of claims 1 to 11 can be cut, optionally wound up into a roll (100) of disposal tape (110), the disposal tape (110) comprising a plurality of disposal tabs (10, 10', 11, 11', 12, 12', 13, 14, 15, 15', 16, 16') according to any one of claims 1 to 11 in an endless form.

13. Method of making a disposal tab (10, 10', 11, 11', 12, 12', 13, 14, 15, 15', 16, 16') according to any one of claims 1 to 12 the method comprising the steps of
a. Providing a disposal tape (110) according to claim 12, wherein the disposal tape (110) has a main longitudinal extension defining a machine direction, optionally including the step of unwinding the disposal tape (110) from a roll (100) of disposal tape (110) along its main longitudinal extension,
b. Cutting the disposal tape (110) in a direction perpendicular to the machine direction so as to obtain individual disposal tabs (10, 10', 11, 11', 12, 12', 13, 14, 15, 15', 16, 16').

14. A disposable article (200) comprising a disposal tab (10, 10', 11, 11', 12, 12', 13, 14, 15, 15', 16, 16') according to any one of claims 1 to 12 attached to the outer surface (220) of the disposable article (200).

15. A disposal tab (17) for securing a disposable article (200) in a disposal configuration, the tab (17) comprises:
a. a first tab element (20) for attaching the tab (17) onto a disposable article (200),
b. a second tab element (30") configured and adapted to elongate the tab (17) along its major longitudinal extension, comprising two inelastic portions (40, 50) and an intermediate elastic portion (60) therebetween and
wherein the first and second tab elements (20, 30") are connected to each other through a third tab element (90') providing a non-releasable connection (24, 54, 82, 84) between the first and third tab element (20, 90') and between the second and third tab element (30', 90') and - before use - through a releasable connection (24, 24') by arranging the third tab element (90') on the first tab element (20) and by arranging the second tab element (30") on the third tab element (90'), whereby a z-folded configuration is formed,
wherein the non-releasable connection (24, 54, 82, 84) is formed either by one end of the first or second tab element (20, 30") being folded onto itself and attached to the second and third tab element (30", 90') or
by a c-shaped center strip (80) encompassing an end of at least the first, second and/or third tab element (20, 30", 90'),
wherein - before use - the second tab element (30, 30') is detachable from the third tab element (90') and wherein the third tab element (90') is detachable from the first tab element (20) upon use of the tab (17) thereby releasing the z-folded configuration, while the tab elements (20, 20', 30, 30') are still connected through the non-releasable connection (24, 54, 82, 84).
